# EUROPÄISCHE PATENTSCHRIFT

(11) **EP 2 292 298 B1**
(45) Veröffentlichungstag und Bekanntmachung des Hinweises auf die Patenterteilung: **13.06.2012**
(21) Anmeldenummer: 10168809.1
(22) Anmeldetag: 08.07.2010
(51) Int. Cl.: A61N 5/10

(54) **Verfahren zur Bestimmung eines Bestrahlungsplans, Bestrahlungsplanungseinrichtung sowie Bestrahlungsanlage**
Method for determining an irradiation plan, irradiation planning device and irradiation assembly
Procédé de détermination d'un plan de rayonnement, dispositif de planification du rayonnement et installation de rayonnement

(30) Priorität: 07.09.2009 DE 102009040390
(43) Veröffentlichungstag der Anmeldung: 09.03.2011
(73) Patentinhaber: SIEMENS AKTIENGESELLSCHAFT, 80333 München (DE)
(72) Erfinder: Rietzel, Eike, 64331, Weiterstadt (DE)

(56) Entgegenhaltungen:
- WO-A2-2005/035061
- US-A1- 2003 219 098
- US-A1- 2007 003 011
- US-A1- 2007 081 629

## Beschreibung

Die Erfindung betrifft ein Verfahren zur Bestimmung eines Bestrahlungsplans, eine Bestrahlungsplanungseinrichtung sowie eine Bestrahlungsanlage.

Die Partikeltherapie ist ein etabliertes Verfahren zur Behandlung von Gewebe, insbesondere von Tumorerkrankungen. Bestrahlungsverfahren, wie sie in der Partikeltherapie eingesetzt werden, finden jedoch auch in nicht-therapeutischen Gebieten Anwendung. Hierzu gehören beispielsweise Forschungsarbeiten, etwa zur Produktentwicklung, im Rahmen der Partikeltherapie, die an nicht-lebenden Phantomen oder Körpern durchgeführt werden, Bestrahlungen von Materialien, etc.

Hierbei werden geladene Partikel wie z.B. Protonen oder Kohlenstoffionen oder andere Ionen auf hohe Energien beschleunigt, zu einem Partikelstrahl geformt und über ein Hochenergiestrahltransportsystem zu einem oder mehreren Bestrahlungsräumen geführt. In dem Bestrahlungsraum wird das zu bestrahlende Zielvolumen mit dem Partikelstrahl bestrahlt.

Bekannt sind Bestrahlungsverfahren, die unter der Bezeichnung Scan-Verfahren bekannt sind. Bei diesen Verfahren wird ein Partikelstrahl mit einem im Vergleich zum Zielvolumen kleinen Durchmesser sukzessive an eine Vielzahl von Zielpunkten im Zielvolumen gelenkt, das Zielvolumen wird durch den Partikelstrahl "gescannt".

Ein Verfahren gemäß dem Oberbegriff des Anspruchs 1 ist aus der US 2007/003011 bekannt.

Ebenfalls bekannt sind Verfahren der sogenannten "inversen" Bestrahlungsplanung. Bei derartigen Verfahren wird ein zu erreichendes Bestrahlungsziel von einem Anwender vorgegeben, z.B. ein zu bestrahlendes Zielvolumen, zu schonende Organe und eine zu erreichende Solldosis. Anschließend wird ermittelt, wie diese Vorgabe bei einer Bestrahlung umgesetzt werden kann, d.h. wie die Parameter, mit denen ein Bestrahlungsvorgang gesteuert werden kann und die letztlich die Dosisdeposition bewirken, eingestellt werden müssen - z.B. von welcher Richtung aus welcher Dosisanteil wohin im Zielvolumen appliziert werden soll. Die Parameter, die die Dosisbelegung kennzeichnen, hängen in komplexer Weise voneinander ab. Daher wird eine inverse Bestrahlungsplanung üblicherweise mit einem Optimierungsalgorithmus durchgeführt, welche diese Abhängigkeiten berücksichtigt.

Es ist die Aufgabe der Erfindung, ein Verfahren zur Bestrahlungsplanung anzugeben, das eine schnelle Berechnung eines Bestrahlungsplans auch bei komplexen Abhängigkeiten erlaubt. Weiterhin ist es die Aufgabe der Erfindung, eine derartige Bestrahlungsplanungseinrichtung und eine derartige Bestrahlungsanlage anzugeben.

Die Aufgabe der Erfindung wird durch die unabhängigen Ansprüche gelöst. Vorteilhafte Weiterbildungen finden sich in den Merkmalen der abhängigen Ansprüche.

Das erfindungsgemäße Verfahren zur Bestimmung eines Bestrahlungsplans umfasst folgende Schritte:
a) Vorgabe eines zu bestrahlenden Zielvolumens und einer zu erfüllenden Bedingung,
b) Durchführen eines ersten Optimierungsschrittes mit folgenden Teilschritten:
   - Bereitstellen eines ersten Datensatzes, in welchem das Zielvolumen abgebildet ist,
   - Ermitteln eines ersten Parametersatzes für die Bestrahlungsplanung, indem ein erster Optimierungsalgorithmus ausgeführt wird,
      bei welchem der erste Parametersatz hinsichtlich der vorgegebenen Bedingung unter Verwendung des ersten Datensatzes optimiert wird,
c) Durchführung zumindest eines weiteren Optimierungsschrittes mit folgenden Teilschritten:
   - Bereitstellen eines weiteren Datensatzes, welcher eine höhere Auflösung aufweist als ein Datensatz, der in einem zuvor durchgeführten Optimierungsschritt verwendet wurde,
   - Ermitteln eines weiteren Parametersatzes für die Bestrahlungsplanung, indem ein weiterer Optimierungsalgorithmus ausgeführt wird,
      bei welchem der weitere Parametersatz hinsichtlich der vorgegebenen Bedingung unter Verwendung des weiteren Datensatzes und unter Verwendung eines Parametersatzes, der in einem zuvor durchgeführten Optimierungsschritt ermittelt wurde, optimiert wird,
d) Erzeugen eines Bestrahlungsplanungsdatensatzes aus dem weiteren Parametersatz.

Die Erfindung bezieht folglich auf ein Bestrahlungsplanungsverfahren, bei dem mehrere Optimierungsschritte nacheinander ausgeführt werden.

Das zu bestrahlende Zielvolumen und die zu erfüllende Bedingung können von einem Anwender vorgegeben werden. Die zu erfüllende Bedingung kann beispielsweise eine Vorgabe sein, welche die zu erreichende Dosisbelegung des Zielvolumens und andere, zu schonende Volumina charakterisiert.

Der durch die Optimierungsschritte ermittelte und optimierte Parametersatz kann in einem Bestrahlungsplanungsdatensatz hinterlegt werden und dient dazu, eine Bestrahlungsanlage entsprechend so zu steuern, so dass eine entsprechende Bestrahlung des Zielvolumens möglichst gut die vorgegebene Bedingung erfüllt.

Ein derartiger Parametersatz kann beispielsweise die Anzahl der nacheinander durchzuführenden Dosisdepositionen, die jeweiligen Einstrahlrichtungen und/oder die jeweils zu applizierende Dosis umfassen. Bei einem Verfahren zur Bestrahlungsplanung für einen Partikelstrahl, welcher im Scanverfahren appliziert werden soll, bei welchem ein Partikelstrahl sukzessive an mehrere Zielpunkte des Zielvolumens zu steuern ist, kann der Parametersatz Werte umfassen, die die pro Zielpunkt zu applizierende Teilchenzahl kennzeichnen.

Der Parametersatz kann je nach Ausgestaltung der Bestrahlungsanlage direkt oder indirekt - d.h. nach entsprechender Interpretation durch einen Steuerungsalgorithmus und Umwandlung in Steuerungsbefehle - zur Steuerung einer Bestrahlungsanlage verwendet werden.

Die Optimierungsschritte unterscheiden sich davon, dass die Auflösung des Datensatzes, der dem jeweiligen Schritt zu Grunde liegt, sukzessive höher wird und dass damit die Berechnung und die Durchführung der Optimierungsschritte sukzessive komplexer und aufwändiger wird.

Der Parametersatz, der in einem vorhergehenden Optimierungsschritt ermittelt wird, kann noch mit vergleichsweise geringer Rechenzeit ermittelt werden, da die Auflösung des Datensatzes im Vergleich zu späteren Schritten gering ist. Dieser Parametersatz fließt bei dem erfindungsgemäßen Verfahren in den nachfolgenden Optimierungsschritt ein. Der Erfindung liegt die Erkenntnis zu Grunde, dass ein derartig aufgebautes Bestrahlungsplanungverfahren schneller und - trotz mehrerer Optimierungsschritte - mit geringerem Rechenaufwand zum Ziel führt als Verfahren, welche von vornherein einen hoch aufgelösten Datensatz verwenden und das Optimum der Parameterwerte direkt mithilfe des hoch aufgelösten Datensatzes ermitteln.

Die Verwendung des Parametersatzes, der in einem zuvor durchgeführten Optimierungsschritt ermittelt und optimiert worden ist, wird dazu verwendet, den oder die nachfolgenden Optimierungsschritte zu beeinflussen, also zu bahnen und in eine Richtung zu lenken. Der Optimierungsalgorithmus, der im nachfolgenden Optimierungsschritt verwendet wird, wird folglich weniger Iterationen brauchen, um zum Ziel zu gelangen und das Optimum für den Parametersatz zu ermitteln.

Insbesondere können aus dem Parametersatz Startwerte ermittelt werden, die den nachfolgenden Optimierungsschritt einfließen. Diese Startwerte stellen bereits eine gute Näherung der Parameterwerte dar, die es im nachfolgenden Optimierungsschritt zu ermitteln und zu optimieren gilt. Es sind im nachfolgenden Optimierungsschritt weniger Iterationen beim Optimierungsalgorithmus notwendig, um den weiteren Parametersatz zu ermitteln, im Vergleich zu einem Optimierungsalgorithmus, dem diese Startwerte nicht zur Verfügung stehen. Damit kann auch bei einem höher aufgelösten Datensatz der Optimierungsalgorithmus schnell durchgeführt werden, da die Startwerte im Allgemeinen nurmehr geringfügig modifiziert und angepasst werden müssen.

Die in ersten Schritt verwendete Optimierungsalgorithmus und der im weiteren Schritt verwendete weitere Optimierungsalgorithmus können die gleichen oder auch verschiedene Optimierungsalgorithmen sein.

Bei den Optimierungsschritten kann in einer vorteilhaften Ausführungsform eine von dem Zielvolumen absorbierte Dosis ermittelt werden. Insbesondere kann dann die zu erreichende Bedingung eine zu erreichende absorbierte Zieldosis sein. Parameterwerte, welche in einem der Optimierungsschritte zur Ermittlung der absorbierten Dosis verwendet werden, können dabei auf einen höher aufgelösten Datensatz, der in einem der nachfolgenden Optimierungsschritte verwendet wird, extrapoliert werden.

Das Verfahren wirkt sich jedoch besonders dann vorteilhaft aus, wenn bei den Optimierungsschritten eine Wirkung der von dem Zielvolumen absorbierten Dosis, also sozusagen eine effektive Dosis, ermittelt wird. Insbesondere kann dann die zu erreichende Bedingung eine zu erreichende effektive Zieldosis bzw. Wirkung auf das Zielvolumen sein. Parameterwerte, welche in einem der Optimierungsschritte zur Ermittlung der effektiven Dosis verwendet werden, können dabei auf einen höher aufgelösten Datensatz, der in einem der nachfolgenden Optimierungsschritte verwendet wird, extrapoliert werden.

Eine derartige Wirkung des Partikelstrahls wird oftmals durch die so genannte RBW (relative biologische Wirksamkeit) gekennzeichnet. Die Berechnung der Wirkung ist insbesondere bei Partikelstrahlen - insbesondere mit Partikeln, die schwerer sind als Protonen - aufgrund der komplexen Interaktion mit dem Zielvolumen sehr rechenintensiv. Daher wirkt sich das Verfahren, das mit stufenweise höheren Auflösungen arbeitet, hier besonders vorteilhaft aus und kann zu einer deutlichen Verkürzung der Rechenzeit führen. Bei der Berechnung der effektiven Dosis bzw. der Wirkung der von dem Zielvolumen absorbierten Dosis wird insbesondere das von dem Partikelstrahl ortsabhängig erzeugte Teilchenspektrum verwendet.

Der Parametersatz, der bei den Optimierungsschritten ermittelt und optimiert wird, kann weitere Werte umfassen, welche nicht direkt oder indirekt zur Steuerung einer Bestrahlungsanlage verwendet werden, welche jedoch bei der Berechnung der zu deponierenden Dosisverteilung verwendet werden.

Derartige Parameterwerte können beispielsweise das Teilchenspektrum kennzeichnen, das durch den zu applizierenden Partikelstrahl erwartungsgemäß erzeugt wird. Das erzeugte Teilchenspektrum hängt von der Anatomie des Zielvolumens ab und von der Wechselwirkung des Partikelstrahls mit der Anatomie des Zielvolumens und ist im Allgemeinen ortsabhängig, d.h. es ändert sich von Voxel zu Voxel des Datensatzes. Mit einem niedrig aufgelösten Datensatz kann das Teilchenspektrum vergleichsweise schnell berechnet werden, während die Berechnung bei einem höher aufgelöste Datensatz aufgrund der komplexen Interaktion des Partikelstrahls mit dem Zielvolumen zeitaufwändig und rechenintensiv ist.

In einer vorteilhaften Ausführungsform des Verfahrens wird bei den Optimierungsschritten ein durch den zu applizierenden Strahl erzeugtes Teilchenspektrum in Abhängigkeit des Ortes im Zielvolumen berechnet, z.B. mit einer Auflösung, welche der Auflösung des Datensatzes entspricht, der bei dem jeweiligen Optimierungsschritt verwendet wird. Diese Berechnung erfolgt üblicherweise voxelweise. Deshalb nimmt die Berechnung bei einem niedrig aufgelösten Datensatz weniger Zeit in Anspruch als bei einem hoch aufgelösten Datensatz.

Das bei einem der vorhergehenden Optimierungsschritte berechnete Teilchenspektrum kann dann auf den höher aufgelösten Datensatz, der einem nachfolgenden Optimierungsschritt verwendet wird, extrapoliert werden. Aus dem bei einem der Optimierungsschritte berechneten Teilchenspektrum können Startwerte für den Optimierungsalgorithmus bei einem der nachfolgenden Optimierungsschritte ermittelt werden, z.B. durch die Extrapolation. Eine Extrapolation kann vergleichsweise schnell und einfach durchgeführt werden.

Die Anzahl der Voxel ist bei einem Datensatz, der in einem vorhergehenden Optimierungsschritt verwendet wird, geringer als die Anzahl der Voxel eines Datensatzes, der in einem der nachfolgenden Optimierungsschritte verwendet wird. Daraus ergeben sich signifikante Einsparungen in der Optimierungszeit, da die geringere Anzahl der Voxel eine deutlich schnellere Berechnung und Durchführung des Optimierungsalgorithmus erlaubt. Bei dieser Optimierung werden insbesondere die in den Voxeln auftretenden Teilchenspektren, insbesondere die vollständigen Teilchenspektren, berechnet. Diese Teilchenspektren sind im Allgemeinen notwendig, um bei einem Partikelstrahl die Wirkung des Partikelstrahls auf das Zielvolumen zu berechnen, d.h. die effektive Dosis und/oder die relativen biologischen Werte zu berechnen. Wenn diese Teilchenspektren in jedem der Voxel auf eine höhere Auflösung extrapoliert wird, sind bereits sehr gute Startwerte für die weitere Optimierung vorgegeben, die an einem höher aufgelöste Datensatz durchgeführt werden kann. Im nächsten Optimierungsschritt sind daher vergleichsweise wenige Iterationen notwendig, um den Parametersatz weiter zu optimieren.

In einer Ausgestaltung des Verfahrens werden die Datensätze unterschiedlicher Auflösung, die bei den Optimierungsschritten verwendet werden, aus einem einzigen Planungsdatensatz ermittelt. Dies kann beispielsweise ein Planungs-CT sein. Die verschiedenen Datensätze können hieraus berechnet werden, indem jeweils unterschiedlich viele benachbarte Voxel zu einem größeren Voxel zusammengefasst werden, beispielsweise durch Mittelwertbildung.

Die erfindungsgemäße Bestrahlungsplanungseinrichtung umfasst eine Rechnereinheit mit einer Eingabeeinrichtung und einer Ausgabeeinrichtung, wobei die Rechnereinheit zur Durchführung eines Verfahrens nach einem der Ansprüche 1 bis 10 ausgebildet ist.

Die erfindungsgemäße Bestrahlungsanlage, insbesondere Partikeltherapieanlage, weist eine derartige Bestrahlungsplanungseinrichtung und einer Steuerungsvorrichtung zur Steuerung der Partikeltherapieanlage auf, welche Bestrahlungsanlage aufgrund eines nach einem Verfahren nach einem der Ansprüche 1 bis 10 hergestellten Bestrahlungsplanungsdatensatz steuern kann.

Auch wenn sich Ausführungsformen der Erfindung besonders bei Partikeltherapieanlagen vorteilhaft auswirken, können Ausführungsformen der Erfindung auch bei einer Bestrahlung mit Röntgenstrahlen eingesetzt werden.

Die vorstehenden und die nachfolgenden Ausführungen zu Merkmalen, deren Wirkungsweise und deren Vorteile beziehen sich jeweils auf die Vorrichtungskategorie und auf die Verfahrenskategorie, ohne dass dies jeweils explizit erwähnt ist. Die dabei offenbarten Einzelmerkmale können auch in anderen als den gezeigten Kombinationen erfindungswesentlich sein.

Ausführungsformen der Erfindung sowie vorteilhafte Weiterbildungen gemäß den Merkmalen der abhängigen Ansprüche werden anhand der folgenden Zeichnung näher erläutert, ohne jedoch darauf beschränkt zu sein. Es zeigen:
- Fig. 1: eine schematisierte Darstellung einer Partikelthera- pieanlage, in der ein Zielvolumen, umfassend mehrere Zielpunkte, die in Iso-Energieschichten angeordnet sind, bestrahlt wird,
- Fig. 2: eine Darstellung von Verfahrensschritten, die bei ei- ner Ausführungsform des Verfahrens durchgeführt wer- den.

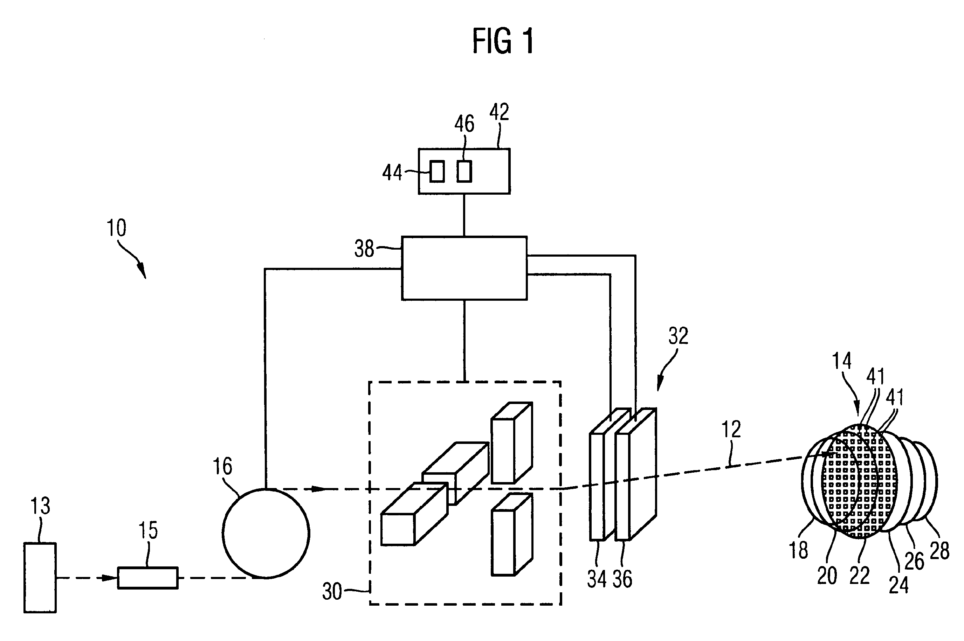

Fig. 1 zeigt in stark schematisierter Darstellung einen Aufbau einer als Partikeltherapieanlage 10 aufgebauten Bestrahlungsanlage. Die Partikeltherapieanlage 10 wird zur Bestrahlung eines üblicherweise mit einer Positioniervorrichtung entsprechend positionierten Zielvolumens mit einem Strahl aus Partikeln, der im Folgenden als Partikelstrahl 12 bezeichnet ist. Insbesondere kann ein tumorerkranktes Gewebe eines Patienten mit dem Partikelstrahl 12 bestrahlt werden. Es ist ebenfalls vorgesehen, die Partikelstrahlanlage 10 zur Bestrahlung eines nicht-lebenden Körpers, insbesondere eines Wasserphantoms oder anderen Phantomen einzusetzen. Die Bestrahlung des Wasserphantoms kann beispielsweise zu Zwecken der Überprüfung und Verifizierung von Bestrahlungsparametern vor und/oder nach einer erfolgten Bestrahlung eines Patienten erfolgen. Es ist aber auch vorgesehen, andere Körper, insbesondere Versuchsaufbauten wie beispielsweise Zellkulturen oder Bakterienkulturen mit dem Partikelstrahl 12 zu bestrahlen.

Die Partikeltherapieanlage 10 weist typischerweise eine Partikelquelle 13 und eine Beschleunigereinheit auf - z.B. ein Synchrotron 16 und einen Vorbeschleuniger 15 oder ein Zyklotron oder einen sonstigen Beschleuniger - die einen Partikelstrahl 12 mit der zur Bestrahlung notwendigen Energie bereitstellt. Als Partikel werden vornehmlich Teilchen wie beispielsweise Protonen, Pionen, Heliumionen, Kohlenstoffionen oder Ionen anderer Elemente eingesetzt. Typischerweise hat einen Partikelstrahl 12 einen Strahldurchmesser von 3-10 mm. Der Partikelstrahl 12 wird zu einem Bestrahlungsraum geführt, in dem sich das Zielvolumen 14 befindet.

In dem zu bestrahlenden Zielvolumen 14 sind Iso-Energieschichten 18, 20, 22, 24, 26 und 28 angedeutet. Eine Iso-Energieschicht 18, 20, 22, 24, 26 oder 28 entspricht jeweils der Eindringtiefe des Braggpeaks für eine bestimmte Energie des Partikelstrahls 12.

Als Scanverfahren wird bevorzugt ein Rasterscan-Verfahren verwendet, bei dem ein Partikelstrahl 12 von Zielpunkt 41 zu Zielpunkt 41 geführt wird ohne zwangsläufige Abschaltung bei einem Übergang von einem Zielpunkt zum nächsten. Es können auch Spotscan-Verfahren mit Abschaltung des Partikelstrahls zwischen den einzelnen Zielpunkten oder andere Scan-Verfahren wie beispielsweise kontinuierliche Scanverfahren eingesetzt werden. In Fig. 1 ist das Scan-Verfahren schematisch illustriert, anhand einiger Zielpunkte 41, welche in einem schichtweise aufgebauten Zielvolumen 14 zum Teil dargestellt sind und welche sukzessive mit dem Partikelstrahl 12 angefahren werden.

Zur Durchführung des Scanverfahrens ist eine Scanvorrichtung 30 mit mehreren Ablenkmagneten, vorzugsweise in zwei orthogonalen Richtungen, vorgesehen, welche es erlaubt, den Partikelstrahl 12 von Zielpunkt 41 zu Zielpunkt 41 zu lenken.

Weiterhin ist eine Strahlmonitoreinrichtung 32 vorgesehen, mit der eine Strahlqualität des Partikelstrahls 12 überprüft werden kann, beispielsweise die durch den Partikelstrahl 12 applizierte Teilchenzahl mit einer Ionisationskammer 34 oder den Ort des Partikelstrahls 12 mit einer Ortsmesskammer 36.

Eine Steuerungseinrichtung 38 steuert die Anlage. So kann die Steuerungseinrichtung 38 beispielsweise den Beschleuniger 15, 16 zur Bereitstellung eines Strahls mit einer gewünschten Intensität steuern, den Strahl gemäß einem Bestrahlungsplan mit der Scanvorrichtung 30 lenken, und die Messdaten der Strahlmonitoreinrichtung 32 zur Überwachung der Strahlqualität auswerten. Weiterhin kann der Steuerungseinrichtung 38 einen von mehreren Messbereichen auswählen, in welchem die Strahlmonitoreinrichtung 32 bzw. deren Messvorrichtungen 34, 36 betrieben werden soll. Die Steuerungseinrichtung 38 ist üblicherweise auf mehrere miteinander vernetzte Untereinheiten aufgeteilt (hier der Übersichtlichkeit halber nicht gezeigt).

Eine Bestrahlungsplanungseinrichtung 42, üblicherweise eine Rechnereinheit, welche eine Eingabeeinrichtung 44 und eine Ausgabeeinrichtung 46 zur Interaktion mit einem Anwender aufweist, ist mit der Steuerungseinrichtung 38 verbunden, so dass ein Bestrahlungsplan, der mit der Bestrahlungsplanungseinrichtung 42 erstellt worden ist, auf der Partikeltherapieanlage 10 ausgeführt werden kann.

Eine derartige Partikeltherapieanlage 10 ist im Stand der Technik bekannt.

In vorteilhafter Weise kann jedoch auf der Bestrahlungsplanungseinrichtung 42 ein Bestrahlungsplan ermittelt werden, wenn eine Ausführungsform des erfindungsgemäßen Verfahrens wie nachfolgend erläutert auf ihr ausgeführt wird.

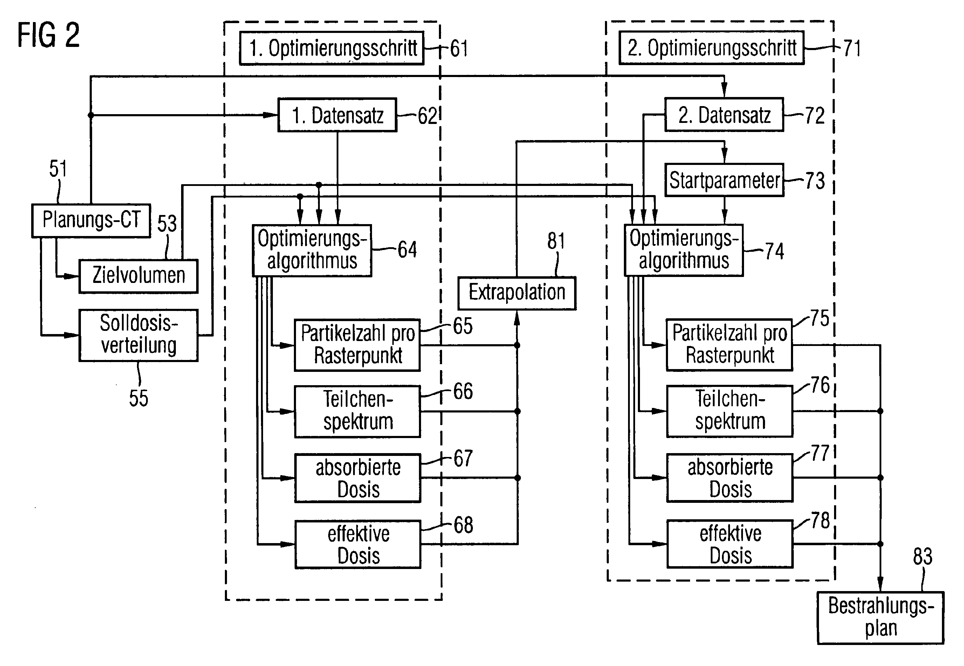

Fig. 2 zeigt eine schematische Übersicht über Verfahrensschritte, die bei einer Ausführungsform des erfindungsgemäßen Verfahrens ausgeführt werden können.

Zu Beginn der Bestrahlungsplanung wird ein Planungs-CT bereitgestellt (Schritt 51). Mithilfe einer Rechnereinheit, die über eine Eingabeeinrichtung (z.B. Maus, Tastatur) und eine Ausgabeeinrichtung (z.B. Monitor) verfügt, kann ein Anwender das zu bestrahlende Zielvolumen markieren (Schritt 53). Falls erforderlich, kann ein Anwender in diesem Schritt auch festlegen, welche Bereiche in dem zu bestrahlenden Objekt von einer Dosisdeposition möglichst ausgespart werden sollen (OAR, für "organs at risk"). Der Anwender gibt eine Soll-Dosisverteilung vor, mit der das Zielvolumen bestrahlt werden soll (Schritt 55).

Anschließend wird ein erster Optimierungsschritt ausgeführt (Schritt 61). Grundlage für diesen ersten Optimierungsschritt bildet ein erster Datensatz, der ähnlich wie das Planungs-CT das Zielvolumen abbildet, wobei der erste Datensatz jedoch eine deutlich geringere Auflösung aufweist als das Planungs-CT (Schritt 62). Der erste Datensatz kann beispielsweise aus dem Planungs-CT erzeugt worden sein.

Der ersten Datensatz bildet Grundlage für einen Optimierungsalgorithmus, mit dem die zur Bestrahlung notwendigen Parameter ermittelt und optimiert werden (Schritt 64).

In den Optimierungsalgorithmus fließen zusätzlich die von dem Anwender vorgenommenen Vorgaben hinsichtlich Zielvolumen und Soll-Dosisverteilung ein. Der Optimierungsalgorithmus kann ein bekannter Optimierungsalgorithmus sein, der bereits im Rahmen der inversen Bestrahlungsplanung eingesetzt wird. Üblicherweise basiert ein derartiger Optimierungsalgorithmus auf einem rekursiven Verfahren.

Mit dem Optimierungsalgorithmus wird ein erster Parametersatz für den Bestrahlungsplan optimiert. Hierzu zählen unter anderem die pro Zielpunkt im Zielvolumen zu applizierende Partikelzahl (Schritt 65), das durch den Partikelstrahl im Zielvolumen erzeugte Teilchenspektrum (Schritt 66), die im Zielvolumen absorbierte Dosis (Schritt 67), und gegebenenfalls die durch den Partikelstrahl im Zielvolumen erzeugte Wirkung (effektive Dosis, Schritt 68).

Mit dem Optimierungsalgorithmus des ersten Optimierungsschrittes werden die Parameter so lange optimiert, bis die Zielvorgabe hinsichtlich der Soll-Dosisverteilung im Zielvolumen möglichst genau erreicht wurde. Da der erste Datensatz jedoch nur eine geringe Auflösung aufweist, werden die Parameter die Erfordernisse noch nicht vollständig erfüllen. Dafür wird eine vergleichsweise geringe Rechenzeit benötigt, um mit dem Optimierungsalgorithmus zu einem ersten Ergebnis für den ersten Parametersatz zu gelangen.

In einem zweiten Optimierungsschritt wird der erste Parametersatz weiter optimiert (Schritt 71).

Hierzu wird aus dem Planungs-CT ein zweiter Datensatz erzeugt, in dem das Zielvolumen ebenfalls abgebildet ist, der aber im Vergleich zum ersten Datensatz eine höhere Auflösung hat (Schritt 72).

In ähnlicher Weise wie bei dem ersten Optimierungsschritt liegt der zweite Datensatz dem Optimierungsalgorithmus des zweiten Optimierungsschrittes zu Grunde. Weiterhin fließen in den Optimierungsalgorithmus des zweiten Optimierungsschrittes die Vorgaben des Anwenders hinsichtlich Zielvolumen und Soll-Dosisverteilung ein (Schritt 74). Aus dem ersten Parametersatz, der im ersten Optimierungsschritt ermittelt wurde, werden Startwerte generiert (Schritt 73), die ebenfalls in den Optimierungsalgorithmus des zweiten Optimierungsschrittes einfließen, und den Ausgangspunkt für die Optimierung darstellen. Da diese Werte bereits eine erste Näherung für die zu optimierenden Parameter darstellen, benötigt der zweite Optimierungsalgorithmus vergleichsweise wenig Zeit und Rechenleistung, um den Parametersatz an den zweiten Datensatz anzupassen und einen zweiten Parametersatz zu finden, der die Vorgaben des Anwenders besser als der erste Parametersatz erfüllt.

Um die Startparameter für den Optimierungsalgorithmus des zweiten Optimierungsschrittes zu finden, kann der erste Parametersatz aus dem ersten Optimierungsschritt auf den zweiten Datensatz extrapoliert werden (Schritt 81).

Der zweite Parametersatz des zweiten Optimierungsschrittes kann analoge Parameter enthalten wie der erste Parametersatz, zum Beispiel die pro Zielpunkt im Zielvolumen zu applizierende Partikelzahl (Schritt 75), das Teilchenspektrum, das durch den Partikelstrahl im Zielvolumen erzeugt wird (Schritt 76), die im Zielvolumen absorbierte Dosis (Schritt 77), sowie die effektive Wirkung des Partikelstrahls im Zielvolumen (Schritt 78).

Falls erforderlich, können ein oder mehrere weiterer Optimierungsschritt analog zum zweiten Optimierungsschritt ausgeführt werden (hier der Übersichtlichkeit halber nicht gezeigt). Dies kann so lange wiederholt und fortgeführt werden, bis die Optimierung an einem Datensatz mit hinreichend genauer Auflösung stattgefunden hat. Aus dem auf diese Weise ermittelten und optimierten Parametersatz kann ein Bestrahlungsplan erstellt werden (Schritt 83).

Aus dem Bestrahlungsplan, üblicherweise ein Datensatz, ist zu entnehmen, wie eine Bestrahlung stattzufinden hat, um die gewünschte Dosisdeposition im Zielvolumen zu erreichen. Dieser Bestrahlungsplan kann von der Steuerungsvorrichtung einer Bestrahlungsanlage eingelesen und interpretiert werden, um die Bestrahlungsanlage zur korrekten Bestrahlung des Zielvolumens zu steuern.

### Bezugszeichenliste

- 10: Partikeltherapieanlage
- 12: Partikelstrahl
- 13: Partikelquelle
- 14: Zielvolumen
- 15: Vorbeschleuniger
- 16: Synchrotron
- 18, 20, 22, 24, 26, 28: Iso-Energieschicht
- 30: Scan-Vorrichtung
- 32: Strahlmonitoreinrichtung
- 34: Ionisationskammer
- 36: Ortsmesskammer
- 38: Steuerungseinrichtung
- 41: Zielpunkte
- 42: Bestrahlungsplanungseinrichtung
- 44: Eingabeeinrichtung
- 46: Ausgabeeinrichtung

- XX: Schritt XX

## Patentansprüche

1. Verfahren zur Bestimmung eines Bestrahlungsplans, umfassend folgende Schritte:
a) Vorgabe eines zu bestrahlenden Zielvolumens (14, 53) und einer zu erfüllenden Bedingung (55),
b) Durchführen eines ersten Optimierungsschrittes (61) mit folgenden Teilschritten:
- Bereitstellen eines ersten Datensatzes (62), in welchem das Zielvolumen (14, 53) abgebildet ist,
- Ermitteln eines ersten Parametersatzes (65, 66, 67, 68) für die Bestrahlungsplanung, indem ein erster Optimierungsalgorithmus (64) ausgeführt wird,
bei welchem der erste Parametersatz (65, 66, 67, 68) hinsichtlich der vorgegebenen Bedingung (55) unter Verwendung des ersten Datensatzes (62) optimiert wird,
c) Durchführung zumindest eines weiteren Optimierungsschrittes (71) mit folgenden Teilschritten:
- Bereitstellen eines weiteren Datensatzes (72), welcher eine höhere Auflösung aufweist als ein Datensatz (62), der in einem zuvor durchgeführten Optimierungsschritt (61) verwendet wurde,
- Ermitteln eines weiteren Parametersatzes (75, 76, 77, 78) für die Bestrahlungsplanung, indem ein weiterer Optimierungsalgorithmus (74) ausgeführt wird,
bei welchem der weitere Parametersatz (75, 76, 77, 78) hinsichtlich der vorgegebenen Bedingung (55) unter Verwendung des zweiten Datensatzes (72) und unter Verwendung eines Parametersatzes (65, 66, 67, 68), der in einem zuvor durchgeführten Optimierungsschritt (61) ermittelt wurde, optimiert wird,
d) Erzeugen eines Bestrahlungsplanungsdatensatzes (83) unter Verwendung des weiteren Parametersatzes (75, 76, 77, 78), **dadurch gekennzeichnet, dass** bei den Optimierungsschritten ein durch den zu applizierenden Strahl erzeugtes Teilchenspektrum (66, 76) in Abhängigkeit des Ortes im Zielvolumen berechnet wird.

2. Verfahren nach Anspruch 1, wobei
bei dem zumindest einen weiteren Optimierungsschritt (71) aus dem Parametersatz (65, 66, 67, 68), der in dem zuvor durchgeführten Optimierungsschritt (61) ermittelt wurde, Startwerte (73) für den weiteren Optimierungsalgorithmus (71) ermittelt werden.

3. Verfahren nach Anspruch 1 oder 2, wobei bei den Optimierungsschritten eine von dem Zielvolumen absorbierte Dosis (67, 77) ermittelt wird.

4. Verfahren nach einem der vorhergehenden Ansprüche, wobei bei den Optimierungsschritten eine Wirkung der von dem Zielvolumen absorbierten Dosis als effektive Dosis (68, 78) ermittelt wird.

5. Verfahren nach einem der vorhergehenden Ansprüche, wobei das bei einem der vorhergehenden Optimierungsschritte berechnete Teilchenspektrum (66) auf den höher aufgelösten Datensatz (72), der einem nachfolgenden Optimierungsschritt (71) verwendet wird, extrapoliert wird.

6. Verfahren nach einem der Anspruch 4 oder 5, wobei aus dem bei einem der Optimierungsschritte (61) berechnete Teilchenspektrum (66) Startwerte (73) für den Optimierungsalgorithmus (74) bei einem der nachfolgenden Optimierungsschritte (71) ermittelt werden.

7. Verfahren nach einem der vorhergehenden Ansprüche, wobei das Verfahren zur Bestrahlungsplanung für einen Partikelstrahl (12), welcher im Scanverfahren zu applizieren ist, durchgeführt wird, bei welchem ein Partikelstrahl sukzessive an Zielpunkte (41) des Zielvolumens (14) zu steuern ist, wobei der Parametersatz Werte (65, 75) umfasst, welche die pro Zielpunkt (41) zu applizierende Partikelzahl charakterisiert.

8. Verfahren nach einem der vorhergehenden Ansprüche, wobei die zu erfüllende Bedingung eine im Zielvolumen (14) zu erreichende Dosisverteilung (55) ist.

9. Verfahren nach einem der vorhergehenden Ansprüche, wobei die Datensätze (62, 72) unterschiedlicher Auflösung, die bei den Optimierungsschritten (61, 71) verwendet werden, aus einem Planungsdatensatz (51) ermittelt werden.

10. Bestrahlungsplanungseinrichtung (42), umfassend eine Rechnereinheit mit einer Eingabeeinrichtung (44) und einer Ausgabeeinrichtung (46), welche zur Durchführung eines Verfahrens nach einem der Ansprüche 1 bis 9 ausgebildet ist.

11. Bestrahlungsanlage, insbesondere Partikeltherapieanlage (10), mit einer Bestrahlungsplanungseinrichtung (42) nach Anspruch 10 und einer Steuerungsvorrichtung (38) zur Steuerung der Bestrahlungsanlage aufgrund eines nach einem Verfahren nach einem der Ansprüche 1 bis 9 hergestellten Bestrahlungsplanungsdatensatzes.

## Claims

1. Method for determining an irradiation plan, comprising the following steps:
a) specifying a target volume (14, 53) to be irradiated and a condition (55) to be fulfilled,
b) implementing a first optimisation step (61) with the following sub-steps:
- providing a first data record (62), in which the target volume (14, 53) is mapped,
- determining a first parameter set (65, 66, 67, 68) for the irradiation planning by a first optimisation algorithm (64) being implemented,
in which the first parameter set (65, 66, 67, 68) is optimised in respect of the predetermined condition (55) by using the first data record (62),
c) implementing at least one further optimisation step (71) with the following sub-steps:
- providing a further data record (72), which has a higher resolution than a data record (62) which was used in a previously implemented optimisation step (61),
- determining a further parameter set (75, 76, 77, 78) for the irradiation planning, by a further optimisation algorithm (74) being implemented,
in which the further parameter set (75, 76, 77, 78) is optimised in respect of the predetermined condition (55) by using the second data record (72) and by using a parameter set (65, 66, 67, 68), which was determined in a previously implemented optimisation step (61),
d) generating an irradiation planning data record (83) by using the further parameter set (75, 76, 77, 78),
**characterised in that**
a particle spectrum (66, 76) generated by the beam to be applied is calculated as a function of the location in the target volume in the optimisation steps.

2. Method according to claim 1, with start values (73) being determined for the further optimisation algorithm (71) in the at least one further optimisation step (71) from the parameter set (65, 66, 67, 68), which was determined in the previously implemented optimisation step (61).

3. Method according to claim 1 or 2, with a dose (67, 77) absorbed by the target volume being determined during the optimisation steps.

4. Method according to one of the preceding claims, with an effect of the dose absorbed by the target volume being determined as an effective dose (68, 78) in the optimisation steps.

5. Method according to one of the preceding claims, with the particle spectrum (66) calculated in one of the preceding optimisation steps being extrapolated onto the higher-resolution data record (72), which is used in a subsequent optimisation step (71).

6. Method according to one of claims 4 or 5, with start values (73) for the optimisation algorithm (74) being determined during one of the subsequent optimisation steps (71) from the particle spectrum (66) calculated in one of the optimisation steps (61).

7. Method according to one of the preceding claims, with the method for irradiation planning for a particle beam (12), which is to be applied in the scanning method, in which a particle beam is to be successively controlled to destinations (41) in the target volume (14), with the parameter set including values (65, 75), which characterises the number of particles to be applied per destination (41).

8. Method according to one of the preceding claims, with the condition to be fulfilled being a dose distribution (55) to be achieved in the target volume (14).

9. Method according to one of the preceding claims, with the data records (62, 72) with a different resolution which are used in the optimisation steps (61, 71) being determined from the planning data record (51).

10. Irradiation planning facility (42), including a computer unit with an input facility (44) and an output facility (46), which is embodied to implement a method according to one of claims 1 to 9.

11. Irradiation system, in particular particle therapy system (10), with an irradiation planning facility (42) according to claim 10 and a control apparatus (38) for controlling the irradiation system on the basis of an irradiation planning data record produced according to a method according to one of claims 1 to 9.

## Revendications

1. Procédé de détermination d'un plan d'irradiation, comprenant les étapes suivantes :
a) prédéfinir un volume cible (14, 53) à irradier et une condition (55) à remplir,
b) effectuer une première étape d'optimisation (61) comportant les étapes partielles suivantes :
- fournir un premier ensemble de données (62) dans lequel le volume cible (14, 53) est représenté,
- déterminer un premier ensemble de paramètres (65, 66, 67, 68) pour la planification d'irradiation, en mettant en oeuvre un premier algorithme d'optimisation (64),
dans lequel ledit premier ensemble de paramètres (65, 66, 67, 68) est optimisé quant à la condition prédéfinie (55) à l'aide du premier ensemble de données (62),
c) effectuer au moins une autre étape d'optimisation (71) comportant les étapes partielles suivantes :
- fournir un autre ensemble de données (72) présentant une résolution plus élevée qu'un ensemble de données (62) ayant été utilisé dans une étape d'optimisation (61) effectuée auparavant,
- déterminer un autre ensemble de paramètres (75, 76, 77, 78) pour la planification d'irradiation, en mettant en oeuvre un autre algorithme d'optimisation (74),
dans lequel ledit autre ensemble de paramètres (75, 76, 77, 78) est optimisé quant à la condition prédéfinie (55) à l'aide du deuxième ensemble de données (72) et à l'aide d'un ensemble de paramètres (65, 66, 67, 68) ayant été déterminé dans une étape d'optimisation (61) effectuée auparavant,
d) créer un ensemble de données de planification d'irradiation (83) à l'aide dudit autre ensemble de paramètres (75, 76, 77, 78),
**caractérisé en ce que** l'on calcule dans les étapes d'optimisation un spectre de particules (66, 76) créé par le faisceau à appliquer, en fonction de l'endroit dans le volume cible.

2. Procédé selon la revendication 1, dans lequel on détermine dans ladite au moins une autre étape d'optimisation (71), à partir dudit ensemble de paramètres (65, 66, 67, 68) qui a été déterminé dans l'étape d'optimisation (61) effectuée auparavant, des valeurs de départ (73) pour l'autre algorithme d'optimisation (71).

3. Procédé selon la revendication 1 ou 2, dans lequel on détermine dans les étapes d'optimisation une dose (67, 77) absorbée par le volume cible.

4. Procédé selon l'une des revendications précédentes, dans lequel on détermine dans les étapes d'optimisation un effet de la dose absorbée par le volume cible comme dose effective (68, 78).

5. Procédé selon l'une des revendications précédentes, dans lequel on extrapole le spectre de particules (66) calculé dans l'une des étapes d'optimisation précédentes, sur l'ensemble de données (72) de résolution plus élevée utilisé dans une étape d'optimisation suivante (71).

6. Procédé selon l'une des revendications 4 ou 5, dans lequel on détermine à partir du spectre de particules (66) calculé dans l'une des étapes d'optimisation (61), des valeurs de départ (73) pour l'algorithme d'optimisation (74) dans l'une des étapes d'optimisation suivantes (71).

7. Procédé selon l'une des revendications précédentes, le procédé de planification d'irradiation étant mis en oeuvre pour un faisceau de particules (12) à appliquer selon le procédé de balayage, dans lequel un faisceau de particules est dirigé successivement sur des points cibles (41) du volume cible (14), ledit ensemble de paramètres comprenant des valeurs (65, 75) qui caractérisent le nombre de particules à appliquer par point cible (41).

8. Procédé selon l'une des revendications précédentes, dans lequel ladite condition à remplir est une distribution de dose (55) à obtenir dans le volume cible (14).

9. Procédé selon l'une des revendications précédentes, dans lequel lesdits ensembles de données (62, 72) de résolution différente utilisés dans les étapes d'optimisation (61, 71) sont déterminés à partir d'un ensemble de données de planification (51).

10. Dispositif de planification d'irradiation (42) comprenant une unité formant ordinateur avec un dispositif d'entrée (44) et un dispositif de sortie (46), lequel est conçu pour la mise en oeuvre d'un procédé selon l'une des revendications 1 à 9.

11. Installation d'irradiation, en particulier installation de thérapie par particules (10), comportant un dispositif de planification d'irradiation (42) selon la revendication 10 et un dispositif de commande (38) pour commander l'installation d'irradiation sur la base d'un ensemble de données de planification d'irradiation créé conformément à un procédé selon l'une des revendications 1 à 9.
